# EUROPÄISCHE PATENTSCHRIFT

(11) **EP 2 547 652 B1**
(45) Veröffentlichungstag und Bekanntmachung des Hinweises auf die Patenterteilung: **03.09.2014**
(21) Anmeldenummer: 11707672.9
(22) Anmeldetag: 10.03.2011
(51) Int. Cl.: C07C 263/10, C07C 265/14

(54) **VERFAHREN ZUR HERSTELLUNG VON ISOCYANATEN**
METHOD FOR PRODUCING ISOCYANATES
PROCÉDÉ DE PRODUCTION D'ISOCYANATES

(30) Priorität: 18.03.2010 EP 10156944
(43) Veröffentlichungstag der Anmeldung: 23.01.2013
(73) Patentinhaber: BASF SE, 67056 Ludwigshafen (DE)
(72) Erfinder: LEHR, Vanessa, Simone, 68199 Mannheim (DE); MATTKE, Torsten, 67251 Freinsheim (DE)
(86) Internationale Anmeldenummer: PCT/EP2011/053583
(87) Internationale Veröffentlichungsnummer: WO 2011/113737

(56) Entgegenhaltungen:
- EP-A1- 1 935 875
- DE-A1- 10 158 160

## Beschreibung

Die Erfindung betrifft ein Verfahren zur Herstellung von Isocyanaten umfassend (a) die Umsetzung von mindestens einem Amin mit Phosgen in der Gasphase in einer Reaktionszone und (b) die anschließende Abkühlung der Reaktionsgase in einer Kühlzone mittels indirekter Kühlung, wobei das die Wärme der Reaktionsgase aufnehmende Kühlmedium zumindest im Bereich der höchsten Temperatur in der Kühlzone im Gegenstrom zum Produktstrom geführt wird.

Isocyanate, insbesondere Diisocyanate, werden überwiegend durch Phosgenierung der entsprechenden Amine hergestellt. Die Phosgenierung kann sowohl in der Flüssigals auch in der Gasphase durchgeführt werden. Die Gasphasenphosgenierung weist in der technischen Umsetzung gegenüber der Flüssigphasenphosgenierung eine Reihe von Vorteilen auf, insbesondere eine höhere Selektivität, ein niedrigerer Hold-Up an toxischem Phosgen sowie niedrigere Investitions- und Energiekosten.

Die Phosgenierung von Aminen zu Isocyanaten in der Gasphase ist an sich bekannt, beispielsweise aus EP-A 570 799, EP-A 749 958 und EP-A 1 078 918. Dabei werden ein aminhaltiger und ein phosgenhaltiger Eduktstrom, sofern sie nicht bereits in der Gasphase vorliegen, verdampft und auf die Reaktionstemperatur der Gasphasenphosgenierung, beispielsweise von etwa 300 bis 400°C, gebracht. Die Bereitstellung der Hochtemperaturwärme nach den bekannten Verfahren ist sehr kostenintensiv.

Der gasförmige Produktstrom muss nach der Reaktion abgekühlt werden. Es ist aus dem Stand der Technik bekannt, dass die Abkühlung der Reaktionsgase schnell erfolgen muss, um die Bildung von unerwünschten Folgeprodukten möglichst zu vermeiden. Im Stand der Technik wird hierfür überwiegend das sogenannte Quenching eingesetzt. Beim Quenching erfolgt die Kühlung mittels direkten Kontaktes mit einer Kühlflüssigkeit, die vorzugsweise in den Strom der heißen Reaktionsgase eingesprüht wird, wie beispielsweise in der EP-A 1 403 248 beschrieben. Hierdurch wird eine schnelle Abkühlung, d. h. eine kurze Abkühlzeit, gewährleistet, so dass die unerwünschte Bildung von Folgeprodukten weitgehend vermieden werden kann.

Aus der EP-A 1 935 875 ist beispielsweise ein Verfahren zur Herstellung von Isocyanaten in der Gasphase bekannt, bei dem zum Abbruch der Reaktion das Reaktionsgemisch aus dem Reaktionsraum heraus durch eine Kühlstrecke geführt wird, in die Flüssigkeiten eingedüst werden, so dass eine direkte Kühlung der Reaktionsgase erfolgt. Die indirekte Kühlung mittels Wärmetauscher ist gemäß dieser Offenlegungsschrift aufgrund des schlechten Wärmeübergangs nachteilig und führt außerdem zur Bildung von Ablagerungen von Feststoffen auf den verhältnismäßig kalten Flächen der Wärmetauscher durch Nebenreaktionen des Gasgemisches auf diesen Flächen. Zur Vermeidung dieser Nachteile schlägt die EP-A 1 935 875 die direkte Kühlung mittels Quenching vor.

Allerdings kommt es gemäß den aus dem Stand der Technik bekannten Kühlverfahren infolge der schnellen Abkühlung der Reaktionsgase und der hohen Temperaturdifferenzen zum Wärme aufnehmenden Medium insbesondere beim Quenching oft zu einer unerwünschten Aerosolbildung. Gebildete Aerosole lassen sich durch herkömmliche technische Verfahren nur schwer wieder abtrennen und erhöhen den Aufwand der weiteren Aufarbeitung zur Aufreinigung der einzelnen Komponenten.

Darüber hinaus lässt sich die Abwärme, die in den direkten Kühlverfahren des Standes der Technik durch Erwärmen oder Verdampfen der Quenchflüssigkeit aufgenommen wird, nur auf tieferem Temperaturniveau nutzen. Werden indirekte Wärmeüberträger genutzt, so ist prinzipiell eine weitere Nutzung der Abwärme möglich. In diesem Falle wäre es wünschenswert, das Wärmeträgermedium, welche die Wärme aufnimmt, soweit zu erhitzen, dass ein hohes Temperaturniveau erreicht wird, welches idealerweise ganz oder annähernd der Temperatur der eingesetzten Eduktströme in der Reaktionszone oder der Verdampfungstemperatur eines oder mehrerer Edukte entspricht. Hierdurch könnte die Wärme auf einem hohen Temperaturniveau gespeichert werden. Dies ermöglicht die Abwärme im Prozess universell wieder einzusetzen.

Es war daher Aufgabe der Erfindung, ein Verfahren zur Herstellung von Mono- und/oder Diisocyanaten durch Gasphasenphosgenierung der entsprechenden Amine zur Verfügung zu stellen, das die vorgenannten Nachteile nicht oder in geringerem Umfang aufweist.

Insbesondere sollte die Bildung von Folgeprodukten vermieden werden. Außerdem sollte die auf das Kühlmedium übertragene Energie auf möglichst hohem Temperaturniveau gehalten werden. Darüber hinaus sollte die Bildung von Aerosolen in der Kühlzone vermieden werden.

Die vorgenannten Aufgaben werden gelöst durch das erfindungsgemäße Verfahren. Bevorzugte Ausführungsformen sind den Ansprüchen und der nachfolgenden Beschreibung zu entnehmen. Kombinationen bevorzugter Ausführungsformen verlassen den Rahmen der vorliegenden Erfindung nicht.

Das erfindungsgemäße Verfahren zur Herstellung von Isocyanaten umfasst zumindest folgende Schritte:
(a) die Umsetzung von mindestens einem Amin mit Phosgen in der Gasphase in einer Reaktionszone und
(b) die anschließende Abkühlung der Reaktionsgase in einer Kühlzone mittels indirekter Kühlung, wobei das die Wärme der Reaktionsgase aufnehmende Kühlmedium zumindest im Bereich der höchsten Temperatur in der Kühlzone im Gegenstrom zum Produktstrom geführt wird.

Die einzelnen Schritte des erfindungsgemäßen Verfahrens werden nachfolgend erläutert.

### Schritt (a)

Unter Reaktionszone ist der Bereich innerhalb des Reaktors zu verstehen, in dem die Umsetzung gemäß Schritt (a) abläuft. Der Bereich oder die Stelle, an dem oder an der die Umsetzung der Ausgangsverbindungen und Zwischenprodukte gemäß der vorliegenden Stöchiometrie und Reaktionskinetik im Wesentlichen abgeschlossen ist, kennzeichnet das Ende der Reaktionszone (räumlich) und das Ende der Umsetzung (reaktionstechnisch). Die Umsetzung der Ausgangsverbindungen ist im Wesentlichen abgeschlossen, wenn die Bildung von mindestens 90 Gew.-%, vorzugsweise mindestens 95 Gew.-%, insbesondere mindestens 99 Gew.-% des mit dem Verfahren hergestellten Isocyanates bzw. den korrespondierenden Carbamoylchloriden abgeschlossen ist.

Im Rahmen des erfindungsgemäßen Verfahrens erfolgt die Umsetzung von Phosgen mit mindestens einem Amin in der Gasphase. Dem Fachmann ist bekannt, dass die Bildung der Isocyanate über Zwischenprodukte abläuft. Zwischenprodukte sind dabei beispielsweise die aus den Diaminen gebildeten Monoamino-monocarbamoylchloride, Dicarbamoylchloride, Monoamino-monoisocyanate und Monoisocyanatomonocarbamoylchloride sowie die Hydrochloride der Aminoverbindungen.

Unter Umsetzung in der Gasphase ist zu verstehen, dass die Edukte und Zwischenprodukte im gasförmigen Zustand miteinander reagieren und im Verlauf der Reaktion während des Durchgangs durch die Reaktionszone zu mindestens 95 Gew.-%, bevorzugt zu mindestens 98 Gew.-%, besonders bevorzugt zu mindestens 99 Gew.-%, ganz besonders bevorzugt zu mindestens 99,5 Gew.-%, insbesondere zu mindestens 99,9 Gew.-% in der Gasphase bleiben.

Die Erfindung ist darüber hinaus an sich nicht eingeschränkt bezüglich der konkreten Durchführung der Umsetzung von Aminen mit Phosgen in der Gasphase (Schritt a). Entsprechende Realisierungen der Umsetzung von Aminen mit Phosgen in der Gasphase gemäß Schritt (a) sind dem Fachmann weitläufig bekannt und beispielsweise in der EP-A 2 062 876 und in den dort zitierten Dokumenten beschrieben. Die vorliegende Erfindung lässt sich somit in bekannte Verfahren zur Herstellung von Isocyanaten durch Phosgenierung von Aminen in der Gasphase integrieren.

Bevorzugte Realisierungen der erfindungsgemäßen Umsetzung gemäß Schritt (a) werden beispielsweise in der WO 2009/027232, WO 2009/037179, WO 2008/086922, EP-A 09167604.9, WO 2010/010135 und WO 2009/027234 beschrieben, auf deren Inhalt hiermit ausdrücklich Bezug genommen wird, wobei jeweils die in den genannten Dokumenten beschriebene Kühlung durch die erfindungsgemäße Kühlung (b) zu ersetzen ist.

Vorzugsweise wird die Umsetzung der Amine, insbesondere Diamine, mit Phosgen im stöchiometrischen Überschuss durchgeführt, so dass die Amine im stöchiometrischen Unterschuss in Bezug auf Phosgen eingesetzt werden. Üblicherweise liegt ein molares Verhältnis von Phosgen zu reaktiven Aminogruppen von 1,1 :1 bis 20 : 1, bevorzugt von 1,2 :1 bis 10 :1 vor.

Der Amine enthaltende Eduktstrom enthält die dem gewünschten Isocyanat-Zielprodukt entsprechenden Amine. Für das erfindungsgemäße Verfahren können solche Amine zur Reaktion zu den korrespondierenden Isocyanaten eingesetzt werden, die bevorzugt ohne wesentliche Zersetzung, d.h. zu mindestens 95 Gew.-% unzersetzt, bevorzugt zu mindestens 98, besonders bevorzugt zu mindestens 99, ganz besonders bevorzugt zu mindestens 99,5, insbesondere zu mindestens 99,8, speziell zu mindestens 99,9 und sogar zu mindestens 99,95 Gew.-% unzersetzt, in die Gasphase überführt werden können. Es können aliphatische, cycloaliphatische oder aromatische Amine, bevorzugt aliphatische oder aromatische Amine eingesetzt werden. Diamine sind als Amine bevorzugt. Isocyanate, die mit dem erfindungsgemäßen Verfahren hergestellt werden können, können demzufolge aromatische, cycloaliphatische oder aliphatische Isocyanate sein, wobei Diisocyanate bevorzugt sind.

Besonders bevorzugt sind Amine, insbesondere Diamine, auf Basis von aromatischen Kohlenwasserstoffen mit von 6 bis 18 Kohlenstoffatomen sowie solche auf Basis von aliphatischen oder cycloaliphatischen Kohlenwasserstoffen mit 2 bis 18 Kohlenstoffatomen. Bevorzugte (cyclo)aliphatische Amine leiten sich von den nachstehend ausgeführten (cyclo)aliphatischen Isocyanaten ab. (Cyclo)aliphatische Isocyanate steht im Rahmen dieser Anmeldung kurz für cycloaliphatische und/oder aliphatische Isocyanate.
Im Folgenden wird auf die bevorzugt erhältlichen Isocyanate Bezug genommen. Die entsprechend bevorzugt eingesetzten Amine gemäß der vorliegenden Erfindung sind diejenigen, die sich durch Ersatz der Isocyanatgruppen durch primäre Amingruppen von den im Folgenden genannten Isocyanaten ableiten.

Cycloaliphatische Isocyanate sind solche, die mindestens ein cycloaliphatisches Ringsystem enthalten.

Aliphatische Isocyanate sind solche, die ausschließlich Isocyanatgruppen aufweisen, die an gerade oder verzweigte Ketten gebunden sind. Diese können auch aromatische Ringsysteme enthalten, solange an diese nicht Isocyanatgruppen gebunden sind.

Aromatische Isocyanate sind solche, die mindestens eine an mindestens ein aromatisches Ringsystem gebundene Isocyanatgruppe aufweisen.

Bevorzugte aromatische Isocyanate enthalten von 6 bis 20 C-Atome. Insbesondere bevorzugt ist monomeres Methylen-di(phenylisocyanat), auch Diisocyanatodiphenylmethan genannt und mit MDI abgekürzt, insbesondere 4,4'-Diisocyanatodiphenylmethan, 2,4'-Diisocyanatodiphenylmethan und/oder 2,2'-Diisocyanatodiphenylmethan, sowie 2,4- und/oder 2,6-Toluylendiisocyanat (TDI) und Naphtyldiisocyanat (NDI).

Bevorzugte (cyclo)aliphatische Diisocyanate sind die aliphatischen Isocyanate Tetramethylendiisocyanat, Pentamethylendiisocyanat (1,5-Diisocyanatopentan), Hexamethylendiisocyanat (1,6-Diisocyanatohexan), 2-Methylpentan-1,5-diisocyanat, 1,8-Octamethylendiisocyanat, 1,10-Decamethylendiisocyanat, 1,12-Dodecamethylendiisocyanat, 1,14-Tetradecamethylendiisocyanat, Derivate des Lysindiisocyanates, Tetramethylxylylendiisocyanat (TMXDI), Trimethylhexandiisocyanat oder Tetramethylhexandiisocyanat, sowie 3 (bzw. 4), 8 (bzw. 9)-Bis(isocyanatomethyl)-tricyclo-[5.2.1.0^{2.6}]decan-Isomerengemische, sowie die cycloaliphatischen Diisocyanate 1,4-, 1,3- oder 1,2-Diisocyanatocyclohexan, 4,4'- oder 2,4'-Di(isocyanatocyclohexyl)methan, 1-Isocyanato-3,3,5- trimethyl-5-(isocyanatomethyl)cyclohexan (Isophorondiisocyanat), 1,3- oder 1,4-Bis(isocyanatomethyl)cyclohexan, 2,4-, oder 2,6-Diisocyanato-1-methylcyclohexan.

Besonders bevorzugt sind 1,6-Diisocyanatohexan, 1-Isocyanato-3,3,5- trimethyl-5-(isocyanatomethyl)cyclohexan, 4,4'-Di(isocyanatocyclohexyl)methan und Toluylendiisocyanat-Isomerengemische.

Außer Diisocyanaten können mit dem erfindungsgemäßen Verfahren prinzipiell auch Monoisocyanate mit einer Isocyanatgruppe oder höhere Isocyanate mit im Mittel mehr als 2 Isocyanatgruppen erhalten werden. Dafür eignen sich beispielsweise Triisocyanate wie Triisocyanatononan, 2,4,6-Triisocyanatotoluol, Triphenylmethantriisocyanat oder 2,4,4'-Triisocyanatodiphenylether oder die Gemische aus Di-, Tri- und höheren Polyisocyanaten, die beispielsweise durch Phosgenierung von entsprechenden Anilin/- Formaldehyd-Kondensaten erhalten werden und Methylenbrücken aufweisende Polyphenylpolyisocyanate darstellen.

Ein bevorzugtes Monoisocyanat stellt Phenylisocyanat dar. Bevorzugt ist jedoch die Herstellung von Diisocyanaten. Ganz besonders bevorzugt wird im Rahmen der vorliegenden Erfindung Toluylendiisocyanat (TDI), insbesondere 2,4- und/oder 2,6-Toluylendiisocyanat erhalten.

Bei der Phosgenierung in der Gasphase ist anzustreben, dass sämtliche im Reaktionsverlauf auftretenden Verbindungen, also Edukte (Amin und Phosgen), Zwischenprodukte (insbesondere die intermediär entstehenden Mono- und Dicarbamoylchloride), Endprodukte (Isocyanat, Chlorwasserstoff), sowie gegebenenfalls zudosiertes Inertmedium, unter den Reaktionsbedingungen innerhalb der mindestens einen Reaktionszone in der Gasphase verbleiben. Bevorzugt liegen die Edukte, die Inerte oder Edukt-Inertgasmischung bereits vor der Reaktionszone vollständig in gasförmiger Phase vor. Das bedeutet, dass der Gasstrom keine Tröpfchen an unverdampftem Amin enthält und Kondensationsvorgänge auf dem Weg zwischen Verdampfung und Mischdüse bis zum Abschluss der Reaktionszone durch geeignete Maßnahmen vermieden werden.

Eine Tröpfchenbildung kann insbesondere durch Überhitzung der Ströme und weitere dem Fachmann bekannte technische Maßnahmen, um Kondensation an kalten Stellen zu verhindern, vermieden werden. Besonders bevorzugt sind die Edukte oder Eduktinertgasmischungen vor der Reaktionszone bereits überhitzt. Überhitzt bedeutet, dass die Eduktmischungen über den bei dem jeweiligen Druck sich ergebenden Siedepunkt überhitzt werden. Diese Überhitzung erfolgt auf Temperaturen die im angegeben Bereich für die Reaktionszone liegen.

Sollten die Edukte oder die sich im Reaktionsverlauf bildenden Zwischenprodukte oder die Zielprodukte sich aus der Gasphase, z. B. an der Reaktorwand oder anderen Apparatebauteilen, abscheiden, so kann durch diese Abscheidungen der Wärmeübergang oder die Durchströmung der betroffenen Bauteile unerwünscht verändert werden.

Das Amin weist zu Beginn von Schritt (a) vorzugsweise eine Temperatur im Bereich von 200 bis 450°C auf. Der Druck des zugegebenen Amins liegt dabei vorzugsweise im Bereich zwischen 0,05 bis 5 bar absolut. Die Temperatur des zugegebenen Phosgens liegt vorzugsweise im Bereich von 250 bis 450°C. Hierzu wird das Phosgen üblicherweise vor Zugabe in dem Fachmann bekannter Weise erwärmt. Besonders bevorzugt werden die beiden Eduktströme, das heißt der das Amin enthaltende Eduktstrom sowie der Phosgen enthaltende Eduktstrom auf eine Temperatur im Bereich von ca. 300 bis 450°C vorerhitzt und somit bei dieser Temperatur in Schritt (a) eingesetzt.

Schritt (a) vorausgehend erfolgt vorzugsweise das Vermischen mindestens eines gasförmigen Phosgen enthaltenden Stromes mit mindestens einem gasförmigen Amin enthaltenden Strom und anschließend gemäß Schritt (a) Umsetzung dieses Gemisches bei einer Temperatur von 200 bis 600 °C in der mindestens einen Reaktionszone.

Die Erhitzung des aminhaltigen Eduktstroms auf Reaktionstemperatur kann mehrstufig erfolgen. Dabei können verschiedene Wärmequellen zum Einsatz kommen. Als Wärmequellen stehen übliche Quellen wie Dampf, elektrische Beheizung und Brenngase zur Verfügung. Darüber hinaus kann die im Rahmen von Schritt (b) vom Kühlmedium aufgenommene Wärme vorteilhaft mindestens teilweise zur indirekten Wärmeübertragung auf mindestens einen Eduktstrom der Umsetzung gemäß Schritt (a), insbesondere auf den aminhaltigen Eduktstrom, genutzt werden.

Die Übertragung der Wärme auf den aminhaltigen Eduktstrom erfolgt grundsätzlich durch indirekte Wärmeübertragung. Gegebenenfalls ist ein separater Wärmeträgerkreis zwischen Wärmequelle und aminhaltigem Eduktstrom installiert. Als Wärmeträger geeignet sind Salzschmelzen oder andere Hochtemperatur-Wärmeträger.

Die Erhitzung des aminhaltigen Eduktstroms umfasst im Wesentlichen eine Erwärmung bis zur Siedetemperatur des aminhaltigen Eduktstroms, dessen Verdampfung und Überhitzung auf die gewünschte Eintrittstemperatur in die Reaktionszone. Jeder dieser drei Schritte kann in einem oder mehreren Apparaten mit identischen oder verschiedenen Wärmequellen durchgeführt werden.

In einer bevorzugten Ausführungsform erfolgt in einem oder mehreren Apparaten eine Erhitzung des aminhaltigen Eduktstroms mit Dampf bis nahe der Kondensationstemperatur des Dampfes bzw. Dampfeintrittstemperatur. Die bis zur gewünschten Eintrittstemperatur in die Reaktionszone gegebenenfalls fehlende Wärmemenge, die zur Überhitzung und gegebenenfalls Verdampfung (bzw. Erhitzung bis auf Verdampfungstemperatur) erforderlich ist, wird vorzugsweise aus der im Rahmen von Schritt (b) vom Kühlmedium aufgenommene Wärme, gegebenenfalls ergänzt durch Wärme aus elektrischer Beheizung oder Brenngasen, bereitgestellt. Dies kann wiederum in einem einzigen oder in mehreren Apparaten erfolgen.

Zur Vorerhitzung der Eduktströme kommen die dem Fachmann bekannten Apparatetypen in Betracht. Zur Vorerhitzung bzw. Überhitzung können insbesondere RohrbündelWärmetauscher, Platten-Wärmetauscher und analoge, dem Fachmann bekannte Wärmetauscher eingesetzt werden.

Die Verdampfung kann in üblichen Verdampfertypen wie Umlaufverdampfern, Fallfilmverdampfern, Kletterfilmverdampfern, Dünnfilmverdampfern, Milli- oder Mikroverdampfern durchgeführt werden. Zur Vermeidung von Zersetzungen kann die Verdampfung bei kleineren Drücken und mithin niedrigeren Siedetemperaturen durchgeführt werden. Eine gegebenenfalls erforderliche Verdichtung auf dem Reaktionsdruck kann durch geeignete Verdichterkonstruktionen oder durch Einsatz einer Ejektordüse als Mischeinrichtung realisiert werden.

Für den phosgenhaltigen Eduktstrom gilt im Wesentlichen das vorangehend für den aminhaltigen Eduktstrom geschriebene. Es kann jedoch sein, dass der zur Verfügung gestellte Phosgenstrom bereits gasförmig vorliegt und damit eine Verdampfung entfällt. Unter Umständen kann ein flüssiger phosgenhaltiger Eduktstrom noch Teile eines höhersiedenden Lösungsmittels enthalten, die in der Reaktionszone unerwünscht sind. In diesem Fall kann eine Teilverdampfung dieses Eduktstroms erfolgen, wobei der lösungsmittelverarmte Gasstrom gegebenenfalls weiter überhitzt als Eduktstrom für die Reaktion dient. Der verbleibende lösungsmittelangereicherte Strom kann einer geeigneten Aufarbeitung zugeführt werden.

Sowohl der Phosgen als auch der das Amin enthaltende Eduktstrom können jeweils mit einem Inertgas verdünnt sein, d. h. im Rahmen der vorliegenden Erfindung kann dem Phosgenstrom und/oder Aminstrom ein zusätzliches Inertmedium beigemischt werden. Bei dem Inertmedium handelt es sich um ein Medium, das bei der Reaktionstemperatur gasförmig im Reaktionsraum vorliegt und im Wesentlichen nicht mit den im Reaktionsverlauf auftretenden Verbindungen reagiert bzw. unter den Reaktionsbedingungen stabil ist. Bevorzugt sind solche Inertmedien, die unter den Reaktionsbedingungen zu mindestens 95 Gew.-% unzersetzt und unreagiert bleiben, bevorzugt zu mindestens 98, besonders bevorzugt zu mindestens 99, ganz besonders bevorzugt zu mindestens 99,5 und insbesondere zu mindestens 99,9 Gew.-%.

Das Inertmedium wird im Allgemeinen vor der Umsetzung mit dem Amin und/oder Phosgen vermischt oder kann schon Bestandteil des rückgeführten Phosgens sein, kann aber auch getrennt von den Eduktströmen beispielsweise direkt in die Reaktionszone dosiert werden. Beispielsweise können Stickstoff, Kohlenstoffdioxid oder Kohlenstoffmonoxid, Edelgase, wie Helium oder Argon, oder Aromaten, wie Toluol oder Xylol oder chlorierte Aromaten, wie Chlorbenzol oder Dichlorbenzol, verwendet werden. Bevorzugt wird Stickstoff und/oder Chlorbenzol als Inertmedium verwendet.

Im Allgemeinen wird das Inertmedium in einer Menge eingesetzt, so dass das Verhältnis der Gasvolumina von Inertmedium zu Amin bzw. zu Phosgen mehr als 0,0001 bis 30, bevorzugt mehr als 0,001 bis 15, besonders bevorzugt mehr als 0,001 bis 5 beträgt.

Die Zuführung von Phosgen über den phosgenhaltigen Strom zum Reaktionsraum kann auch so erfolgen, dass man statt eines einzelnen phosgenhaltigen Stroms mehrere phosgenhaltige Teilströme zuführt. In einem solchen Fall werden die phosgenhaltigen Teilströme zu einem phosgenhaltigen Gesamtstrom addiert.

In einer möglichen Ausführungsform wird das eingesetzte Phosgen und/oder das im Prozess zurückgeführte Phosgen nicht direkt der Reaktionszone, sondern zunächst einer Phosgenreinigung zugeführt. Dies kann insbesondere sinnvoll sein, um dort Spuren von molekularem Chlor abzutrennen, so dass diese Ausführungsform insbesondere dann bevorzugt ist, wenn das aus der Phosgensynthese erhaltene Phosgen noch Spuren von molekularem Chlor (Cl₂) enthält, beispielsweise bei einem Gehalt von mehr als 5 ppm, bevorzugt bei mehr als 3 ppm, besonders bevorzugt bei mehr als 1 ppm, ganz besonders bevorzugt bei mehr als 0,5 ppm und insbesondere bei einem Gehalt von mehr als 0,1 ppm.

Die Vermischung der Reaktanden erfolgt vorzugsweise in einer Mischvorrichtung, die sich vorzugsweise durch eine hohe Scherung der durch die Mischvorrichtung geführten Reaktionsströme auszeichnet. Bevorzugt werden als Mischvorrichtung eine statische Mischvorrichtung wie eine Mischdüse verwendet, die der Reaktionszone gemäß Schritt (a) des erfindungsgemäßen Verfahrens vorangestellt ist. Besonders bevorzugt wird eine Mischdüse verwendet.

Die Mischung kann durch unterschiedliche Maßnahmen erfolgen, insbesondere wie beschrieben in EP-A 699 657, EP-A 1 319 655, Sp. 1, Z. 54 bis Spalte 2, Zeile 24 und Spalte 4, Zeile 16 bis 40, EP-A 1 275 640, Spalte 3, Zeile 27 -bis Spalte 4, Zeile 5, EP-A 1 362 847, Spalte 2, Zeile 19 bis Spalte 3, Zeile 51 und Spalte 4, Zeile 40 bis Spalte 5, Zeile 12 und WO 2010/010135, auf die jeweils im Umfang dieser Offenbarung ausdrücklich Bezug genommen sei.

Nach dem Vermischen in der Mischvorrichtung wird das gasförmige Gemisch aus Phosgen, Amin und gegebenenfalls Inertmedium in den Reaktor geführt, wobei der Reaktor die Reaktionszone umfasst.

Unter Reaktor wird die technische Vorrichtung verstanden, die die Reaktionszone enthält. Im Rahmen von Schritt (a) kommen alle üblichen, aus dem Stand der Technik bekannten Reaktoren in Betracht, die zur nicht katalytischen, einphasigen Gasreaktion, bevorzugt zur kontinuierlichen nicht katalytischen, einphasigen Gasreaktion, geeignet sind und die den geforderten Drücken standhalten. Geeignete Materialien für den Kontakt mit dem Reaktionsgemisch sind z. B. Metalle, wie Stahl, insbesondere legierter Stahl, Tantal, Nickel, Nickellegierungen, Silber oder Kupfer, Glas, Keramik, Emaille oder homogene oder heterogene Gemische und Bauteile daraus. Bevorzugt werden Stahlreaktoren verwendet. Die Wände des Reaktors können glatt oder profiliert sein. Als Profile eignen sich beispielsweise Ritzen oder Wellen.

Entsprechende Reaktorbautypen sind bekannt. Beispiele für geeignete Reaktoren sind in der EP-B1 289 840, Sp. 3, Z. 49 - Sp. 4, Z. 25, in EP-B1 593 334, WO 2004/026813, S. 3, Z. 24 - S. 6, Z. 10, in WO 03/045900, S. 3, Z. 34 - S. 6, Z. 15, in EP-A1 1 275 639, Sp. 4, Z. 17 - Sp. 5, Z. 17 und in der EP-B1 570 799, Sp. 2, Z. 1 - Sp. 3, Z. 42, beschrieben, auf die jeweils im Umfang dieser Offenbarung ausdrücklich Bezug genommen sei. Bevorzugt verwendet werden Rohrreaktoren.

Ebenfalls ist es möglich, im Wesentlichen quaderförmige Reaktionsräume, bevorzugt Plattenreaktoren bzw. Plattenreaktionsräume zu verwenden. Ein besonders bevorzugter Plattenreaktor weist ein Verhältnis von Breite zu Höhe von mindestens 2 : 1, bevorzugt mindestens 3 : 1, besonders bevorzugt mindestens 5 : 1 und insbesondere mindestens 10 : 1 auf. Die obere Grenze des Verhältnisses von Breite zu Höhe hängt von der gewünschten Kapazität des Reaktionsraums ab und ist prinzipiell nicht begrenzt. Als technisch sinnvoll haben sich Reaktionsräume mit einem Verhältnis von Breite zu Höhe bis maximal 5000 : 1, bevorzugt 1000 : 1 erwiesen.

Die Umsetzung von Phosgen mit Amin in der Reaktionszone erfolgt bei Absolutdrücken von mindestens 0,1 bar bis höchstens 20 bar, bevorzugt von 0,5 bar bis 10 bar, besonders bevorzugt von 0,7 bar bis 5 bar.

In einer bevorzugten Ausführungsform besteht der Reaktor aus einem Bündel von Reaktoren. In einer möglichen Ausführungsform muss es sich bei der Mischeinheit nicht um eine eigenständige Vorrichtung handeln, vielmehr kann es vorteilhaft sein, die Mischeinheit in den Reaktor zu integrieren. Ein Beispiel einer integrierten Einheit aus Mischeinheit und Reaktor stellt ein Rohrreaktor mit angeflanschten Düsen dar.

Bei dem erfindungsgemäßen Schritt (a) wird die Temperatur in der Reaktionszone vorzugsweise so gewählt, dass sie oberhalb der Dissoziationstemperatur der Hydrochloride des eingesetzten Diamins, bezogen auf die im Reaktionsraum herrschenden Partialdruckverhältnisse, liegt. Je nach eingesetztem Amin und eingestelltem Druck ergibt sich üblicherweise eine vorteilhafte Temperatur im Reaktionsraum von mehr als 200 °C, bevorzugt mehr als 260 °C und besonders bevorzugt mehr als 300 °C. In der Regel beträgt die Temperatur bis zu 600, bevorzugt bis zu 570 °C.

Die mittlere Kontaktzeit des Umsetzungsgemisches im erfindungsgemäßen Schritt (a) beträgt im allgemeinen zwischen 0,001 Sekunden und weniger als 5 Sekunden, bevorzugt von mehr als 0,01 Sekunden bis weniger als 4 Sekunden, besonders bevorzugt von mehr als 0,02 Sekunden bis weniger als 3 Sekunden. Unter mittlerer Kontaktzeit wird die Zeitspanne vom Beginn der Vermischung der Edukte bis zum Verlassen der Reaktionszone in die Kühlzone verstanden. In einer bevorzugten Ausführungsform ist die Strömung im erfindungsgemäßen Verfahren durch eine Bodenstein-Zahl von mehr als 10, bevorzugt mehr als 100 und besonders bevorzugt von mehr als 500 charakterisiert.

In einer bevorzugten Ausführungsform werden die Abmessungen des Reaktors und die Strömungsgeschwindigkeiten so gewählt, dass eine turbulente Strömung, d.h. eine Strömung mit einer Reynolds-Zahl von mindestens 2300, bevorzugt mindestens 2700, für das Reaktionsgemisch vorliegt, wobei die Reynolds-Zahl mit dem hydraulischen Durchmesser des Reaktionsraumes gebildet wird.

Bevorzugt durchlaufen die gasförmigen Reaktionspartner den Reaktionsraum mit einer Strömungsgeschwindigkeit von 3 bis 400 Meter/Sekunde, bevorzugt von 10 bis 250 Meter/Sekunde. Durch die turbulente Strömung werden eine enge Verweilzeit mit geringer Standardabweichung von meist nicht mehr als 6%, wie in EP-A 570 799 beschrieben, und eine gute Vermischung erreicht. Maßnahmen, wie beispielsweise die in EP-A 593 334 beschriebene Verengung, die zudem verstopfungsanfällig ist, sind nicht notwendig.

Um Produktionsanlagen mit hoher Anlagenkapazität zu bauen, können mehrere Reaktorrohre parallel geschaltet werden. Entsprechende Reaktortypen sind dem Fachmann bekannt. Das Reaktionsvolumen, d. h. die Reaktionszone im Reaktor, kann über seine Außenfläche temperiert werden und die Reaktion isotherm geführt werden. Die Umsetzung kann aber auch adiabat erfolgen. Bei der adiabaten Reaktionsführung findet kein Wärmeaustausch mit der Umgebung statt. Dies kann insbesondere durch thermische Isolierung der Reaktionszone bzw. der Reaktorrohre erfolgen. Bevorzugt findet die Umsetzung adiabat statt. Es sind jedoch auch Zwischenstufen von ideal adiabater und ideal isothermer Reaktionsführung möglich, in denen die oben genannte Isolierung so ausgeführt ist, dass noch teilweise ein Wärmeaustausch mit der Umgebung stattfindet oder optional eine Begleitbeheizung angebracht wird, um Wärmeverluste auszugleichen.

Die Umsetzung gemäß Schritt (a) des erfindungsgemäßen Verfahrens wird bevorzugt einstufig durchgeführt. Darunter ist zu verstehen, dass die Vermischung und Umsetzung der Edukte in einem Schritt innerhalb des gewünschten Temperaturbereiches erfolgt. Ferner wird das erfindungsgemäße Verfahren bevorzugt kontinuierlich durchgeführt.

### Schritt (b)

Erfindungsgemäß erfolgt im Rahmen von Schritt (b) die anschließende Abkühlung der Reaktionsgase in einer Kühlzone mittels indirekter Kühlung, wobei das die Wärme der Reaktionsgase aufnehmende Kühlmedium zumindest im Bereich der höchsten Temperatur in der Kühlzone im Gegenstrom zum Produktstrom geführt wird.

Der Begriff "Kühlzone" kennzeichnet im Rahmen von Schritt (b) diejenige Zone, innerhalb der die Reaktionsgase von der Temperatur, bei welcher sie die Reaktionszone verlassen, auf eine Temperatur abgekühlt werden, die unterhalb der genannten Temperatur liegt. Die Kühlzone befindet sich in einer entsprechenden Kühlvorrichtung, die somit als Wärmetauscher fungiert.

Der Begriff "Produktstrom" kennzeichnet den Strom der im Rahmen von Schritt (a) entstehenden gasförmigen Produkte. Letztere werden als Reaktionsgase bezeichnet.

Im Rahmen von Schritt (b) erfährt der Produktstrom somit eine Kühlung. Die Richtung des Produktstromes definiert die Produktstromrichtung. Der Bereich der höchsten Temperatur in der Kühlzone bezieht sich dabei auf die Temperatur des Produktstromes. Die Richtung des Produktstromes ist vorzugsweise über die gesamte Kühlzone gleich bzw. konstant und ergibt sich aus der Geometrie des Apparates, in dem die Kühlung stattfindet. Im Fall einer vorzugsweise rohrförmig ausgelegten Kühlzone ist die Produktstromrichtung durch die Richtung des Rohres bzw. der Rohre gegeben. Die Produktstromrichtung kann sich im Laufe der Kühlzone ändern, beispielsweise durch Umlenkung der Rohre.

Die von den Reaktionsgasen abgegebene Wärme wird im Rahmen der indirekten Kühlung, d. h. ohne direkten Kontakt mit dem Wärmeüberträgermedium in einem Wärmetauscher auf ein Wärmeüberträgermedium übertragen Das Wärmeüberträgermedium wird im Folgenden als Kühlmedium bezeichnet. Das Kühlmedium ist somit unter den gegebenen Temperaturen ein Fluid, vorzugsweise eine Flüssigkeit (Kühlflüssigkeit).

Das Kühlmedium muss kontinuierlich bewegt werden, um die Wärme kontinuierlich abzuführen. Hieraus ergibt sich eine kontinuierliche Bewegung des Kühlmediums relativ zum Produktstrom. Grundsätzlich kann ein Kühlmedium insgesamt oder Teile des Kühlmediums in vom Produktstrom unterschiedliche Richtungen oder in zum Produktstrom gleiche Richtungen geführt werden, beispielsweise im Gleichstrom oder im Gegenstrom zum Produktstrom.

Der Begriff "im Gegenstrom" bedeutet im Rahmen der vorliegenden Erfindung, dass in Richtung des Produktstromes an einer ersten Stelle x das im Gegenstrom geführte Kühlmedium die Temperatur T_{K}(x) und an einer zweiten Stelle x*, die produktstromabwärts, d. h. in Fließrichtung des Produktstromes liegt, die Temperatur T_{K}(X*) aufweist, wobei T_{K}(x*) kleiner T_{K}(x) ist. Da der gleiche Zusammenhang auch für die Temperatur des Produktstromes T_{P} gilt, bedeutet "im Gegenstrom", dass die Temperatur des Kühlmediums T_{K} in Produktstromrichtung abnimmt.

Es ist grundsätzlich auch denkbar, den Strom des Kühlmediums in ein oder mehrere Teilströme aufzuteilen und mehrere der vorgenannten Stromrichtungen zu realisieren.

Erfindungsgemäß wird jedoch Kühlmedium, d. h. zumindest ein Teilstrom des Kühlmediums, zumindest im Bereich der höchsten Temperatur in der Kühlzone im Gegenstrom zum Produktstrom geführt. Vorzugsweise wird der gesamte Strom des Kühlmediums im Rahmen von Schritt (b) zumindest im Bereich der höchsten Temperatur in der Kühlzone in Gegenstromrichtung zum Produktstrom geführt. Vorzugsweise wird jedoch mindestens ein Teilstrom und insbesondere das Kühlmedium insgesamt in einem Bereich, der für mindestens 30%, vorzugsweise mindestens 50% der aus Schritt (b) resultierenden Temperaturerniedrigung verantwortlich ist, in Gegenstromrichtung zum Produktstrom geführt. Besonders bevorzugt wird der Strom des Kühlmediums in der gesamten Kühlzone gemäß Schritt (b) in Gegenstromrichtung zum Produktstrom geführt.

Der Gegenstrom kann auf verschiedene Arten realisiert werden, beispielsweise durch Stromführung parallel zum Produktstrom, wobei das Kühlmedium in Gegenstromrichtung fließt, oder in einer gekreuzten Stromführung, wobei sich der Produktstrom und der Strom des Kühlmediums an bestimmten Stellen kreuzen. Der Strom des Kühlmedium kann alternativ spiralförmig um den Produktstrom herum angeordnet sein. Es sind auch Zwischenstufen der vorgenannten Realisierungen denkbar.

In einer ersten bevorzugten Ausführungsform wird das Kühlmedium in Gegenstromrichtung parallel zum Produktstrom geführt (Winkel 180°). Dies kann insbesondere durch eine mantelförmige Anordnung des Stromes des Kühlmediums um das oder um die Produktstromrohre herum realisiert werden.

In einer zweiten bevorzugten Ausführungsform wird das Kühlmedium so geführt, dass es spiralförmig entlang des Produktstromes in Gegenstromrichtung geführt wird.

In einer dritten, besonders bevorzugten Ausführungsform wird das Kühlmedium gekreuzt geführt. Unter gekreuzter Führung ist zu verstehen, dass der Strom des Kühlmediums den Strom des Produktstromes an mindestens zwei Stellen kreuzt, wobei die globale Fließrichtung des Kühlmediums so gewählt wird, dass das Kühlmedium in Gegenstromrichtung zum Produktstrom strömt. Diese Ausführungsform lässt sich insbesondere realisieren, indem das Kühlmedium die Rohre, in denen sich der Produktstrom bewegt (Produktrohre), an einer ersten Kontaktstelle kreuzt, wobei das Kühlmedium dann umgelenkt wird und die Produktrohre stromabwärts in Bezug auf den Kühlstrom und stromaufwärts in Bezug auf den Produktstrom weitere male kreuzt. An den Kontaktstellen selbst kann der Winkel der Richtung des Kühlstromes relativ zur Produktstromrichtung 90° betragen oder von 90° abweichen, insbesondere von 60° bis 150°, vorzugsweise von mehr als 70° bis 140°. Die Umlenkung des Kühlstroms kann dabei im Bereich der Produktrohre, d. h. an oder nahe an den Produktrohren, oder außerhalb dieses Bereiches erfolgen. Ganz besonders bevorzugt erfolgt die Abkühlung der Reaktionsgase gemäß Schritt (b) in einem Rohrbündelwärmeübertrager. Rohrbündelwärmeübertrager sind dem Fachmann auch als Rohrbündelwärmetauscher bekannt und ermöglichen die beschriebene gekreuzte Führung des Kühlmediums. Die Begriffe Rohrbündelwärmeübertrager und Rohrbündelwärmetauscher sind im Rahmen der vorliegenden Erfindung synonym zu verstehen.

Ziel des erfindungsgemäßen Schrittes (b) ist die ausreichend schnelle Absenkung der Temperatur der Produkte auf eine Temperatur, bei der die Bildung von Neben- bzw. Folgeprodukten vermieden wird. Der Wärmetauscher muss die Abkühlung auf die gewünschte End- oder Zwischentemperatur somit ausreichend schnell ermöglichen. Gleichzeitig ermöglicht die Realisierung des Gegenstromprinzips eine geringe oder verschwindende Temperaturdifferenz zwischen der Temperatur der Reaktionsgase und der Temperatur des Kühlmediums an der Eintrittsstelle der Reaktionsgase in die Kühlzone und im weiteren Bereich der Kühlzone.

Vorzugsweise erfolgt die Abkühlung in der Kühlzone mittels eines Wärmetauschers mit einer spezifischen Wärmeübertragungsfläche von mindestens 300 m² pro m³ Reaktionsgas. Besonders bevorzugt beträgt die spezifische Wärmeübertragungsfläche in der Kühlzone mindestens 350 m² pro m³ Reaktionsgas, insbesondere mindestens 400 m² pro m³ Reaktionsgas, ganz besonders bevorzugt mindestens 450 m² pro m³ Reaktionsgas. Konstruktionsbedingt ist die spezifische Wärmeübertragungsfläche nach oben begrenzt und beträgt beispielsweise höchstens 5000 m² pro m³ Reaktionsgas, insbesondere höchstens 4000 m² pro m³ Reaktionsgas.

Grundsätzlich kommen solche Wärmetauscher in Betracht, die eine Realisierung des Gegenstromprinzips ermöglichen. Geeignete Wärmetauscher sind dem Fachmann an sich bekannt. Vorzugsweise ermöglichen sie die gewünschte spezifische Wärmeübertragungsfläche von mindestens 300 m² pro m³ Reaktionsgas.

Geeignete Wärmetauscher sind insbesondere Rohrbündelwärmetauscher, Platten-wärmetauscher und Spiralwärmetauscher.

Vorzugsweise wird als Wärmetauscher für die indirekte Kühlung ein Rohrbündelwärmetauscher verwendet. Ein Rohrbündelwärmetauscher ermöglicht eine einfache und kostengünstige Realisierung der bevorzugten spezifischen Wärmeübertragungsfläche und des erfindungsgemäßen Gegenstromprinzips.

In einer möglichen Ausführungsform ist die Temperatur am Ende von Schritt (b) kleiner als die Temperatur des Taupunktes des Reaktionsgemisches, so dass das im Reaktionsgemisch enthaltene Isocyanat durch Kondensation zumindest teilweise in die flüssige Phase übergeht, während Phosgen und Chlorwasserstoff im wesentlichen vollständig in der Gasphase verbleiben.

Der Wärmetauscher, innerhalb dessen die indirekte Kühlung erfolgt, kann somit als Kondensator ausgelegt sein. Die indirekte Kühlung kann demzufolge soweit erfolgen, dass das Isocyanat mindestens teilweise in der Kühlzone kondensiert. Die gegebenenfalls erforderliche weitere Abkühlung kann durch nachfolgende weitere Kühlstufen erfolgen (siehe unten). Alternativ können auch abgekühlte, aber nicht kondensierte Reaktionsgase aus Schritt (b) einer weiteren Kühlstufe oder direkt einer Kolonne zur Leichtsiederabtrennung zugeführt werden.

Vorzugsweise wird die indirekte Kühlung im Rahmen von Schritt (b) zunächst bis zu einer solchen Temperatur durchgeführt, dass das Isocyanat und gegebenenfalls im Produktstrom enthaltene Hochsieder im Wesentlichen nicht kondensieren. Hierdurch kann die Bildung von Ablagerungen wirksam verhindert werden.

Vorzugsweise beträgt die Abkühlzeit im Rahmen von Schritt (b) von 0,01 bis 10 Sekunden, vorzugsweise von 0,02 bis 5 Sekunden, insbesondere von 0,05 bis 3 Sekunden. Hierdurch kann die Bildung von Folge- und Nebenprodukten wirksam vermieden werden. Unter Abkühlzeit wird im Rahmen der vorliegenden Erfindung diejenige Zeit verstanden, die benötigt wird, um den Produktstrom von der Temperatur des Eintretens in die Kühlzone bis auf eine Temperatur abzukühlen, die 50°C oberhalb des Taupunktes der Reaktionsgase liegt.

Die Kühlzone gemäß Schritt (b) kann apparativ auf unterschiedliche Weise realisiert werden. Beispielsweise kann die Kühlzone in einem von der Reaktionszone getrennten Apparat realisiert werden. Alternativ kann in einer zweiten Ausführungsform die Kühlzone zusammen mit der Reaktionszone innerhalb eines Apparates untergebracht werden, was bevorzugt ist. Im Fall der zweiten bevorzugten Ausführungsform handelt es sich vorzugsweise um einen rohrförmigen Apparat (Strömungsrohr bzw. Rohrreaktor), wobei der innere Rohrdurchmesser in der Kühlzone höchstens um 50% vom Durchmesser der Reaktionszone abweicht, besonders bevorzugt höchstens 20%, insbesondere den gleichen Durchmesser aufweist. Hierdurch kann eine Störung der Strömung vermieden werden. Insbesondere wird eine gleichmäßige Durchströmung des Rohrbündels ermöglicht, so dass eine gleichmäßige Kühlung erfolgt, was wiederum die Bildung von Folgeprodukten weiter vermindert. Vorzugsweise enthält das genannte Strömungsrohr keine Einbauten, die als Strömungsstörer fungieren.

In einer besonders bevorzugten Ausführungsform wird die durch indirekte Abkühlung übertragene Wärme mindestens teilweise zur indirekten Wärmeübertragung auf mindestens einen Eduktstrom der Phosgenierung in der Gasphase genutzt. Dies wird durch das hohe erreichbare Temperaturniveau des Kühlmediums gemäß Schritt (b) ermöglicht. Verfahren zur indirekten Wärmeübertragung von einem Fluid auf mindestens einen Eduktstrom der Phosgenierung in der Gasphase sind dem Fachmann an sich bekannt. Auf diese Weise lässt sich die in Schritt (b) gewonnene Wärme nutzen und die Energiekosten des Verfahrens erheblich senken.

Als Kühlmedium eignen sich grundsätzlich solche Substanzen, die im erforderlichen Temperaturbereich flüssig und thermisch stabil sind. Vorzugsweise wird zur indirekten Kühlung eine Salzschmelze verwendet. Bevorzugte Salzschmelzen sind Schmelzen von Alkalimetallnitraten, insbesondere Na- und/oder K-Nitrat.

An die Kühlung gemäß Schritt (b) kann sich eine weitere Kühlstufe oder gegebenenfalls mehrere weitere Kühlstufen anschließen.

In einer bevorzugten Ausführungsform schließt sich an die Kühlung gemäß Schritt (b) mindestens eine weitere Kühlstufe (bb) an. Eine zweite (und weitere) Kühlstufe ist dadurch gekennzeichnet, dass im Fall einer direkten Kühlung in der Stufe (bb) das Prinzip der Kühlung unterschiedlich ist oder dass im Fall einer indirekten Kühlung in der Stufe (bb) die jeweiligen Kühlkreisläufe der Schritte (b) und (bb) voneinander getrennt sind.

In der bevorzugten Ausführungsform erfolgt gemäß der erfindungsgemäßen ersten Kühlstufe (b) die Abkühlung der Reaktionsgase in einer ersten Kühlzone, wobei der Produktstrom in der Kühlzone mittels eines Kühlmediums indirekt gekühlt und wie vorstehend beschrieben das die Wärme der Reaktionsgase aufnehmende Kühlmedium zumindest im Bereich der höchsten Temperatur in der Kühlzone im Gegenstrom zum Produktstrom geführt wird. In einer zweiten Stufe (bb) erfolgt anschließend die weitere Abkühlung der Reaktionsgase in einer zweiten Kühlzone. An die Stufe (bb) können sich optional weiterer Kühlzonen und Kühlschritte (bbb usw.) anschließen.

Die zweite Kühlstufe (bb) kann sich von der ersten Kühlstufe (b) konzeptionell unterscheiden oder analog durchgeführt werden. Beispielsweise kann im Rahmen der zweiten Kühlstufe (bb) ebenfalls indirekt gekühlt werden, optional in Gegenstromrichtung wie vorstehend ausgeführt. Alternativ kann im Rahmen der Stufe (bb) direkt gekühlt werden, was bevorzugt ist. Hierdurch lässt sich die Bildung von Ablagerungen weiter verringern.

Wie bereits ausgeführt ist es bevorzugt, wenn der Produktstrom nach Schritt (b) nicht kondensiert vorliegt. Es ist jedoch bevorzugt, wenn der Produktstrom nach Schritt (bb) zumindest teilweise kondensiert vorliegt.

Im Rahmen einer besonders bevorzugten Ausführungsform wird im Rahmen von Schritt (bb) mittels Quenching gekühlt. Das direkte Kühlen mittels Quenching ist dem Fachmann an sich bekannt.

Im Rahmen des Quenching führt man das vorgekühlte Umsetzungsgemisch einer Mischeinrichtung (Quench) zu, in der man die Temperatur des Gases durch Einbringen einer kühleren Flüssigkeit absenkt. Ausführungsformen dieser Verfahrensstufe können sein: Waschtürme, Rührbehälter, Blasensäulen, Quenchdüsen und ähnliches. In ihnen wird durch Kontakt mit mindestens einem, bevorzugt genau einem inerten Lösungsmittel das gebildete Isocyanat aus dem gasförmigen Reaktionsgemisch auskondensiert (ausgewaschen), während überschüssiges Phosgen, Chlorwasserstoff und gegebenenfalls das Inertmedium diese Aufarbeitungsvorrichtung im Wesentlichen gasförmig durchlaufen.

Die Quenchflüssigkeit muss eine gute Löslichkeit für Isocyanate aufweisen. Vorzugsweise werden organische Lösungsmittel eingesetzt. Insbesondere eingesetzt werden aromatische Lösungsmittel, die mit Halogenatomen substituiert sein können. Beispiele für derartige Flüssigkeiten sind Toluol, Benzol, Nitrobenzol, Anisol, Chlorbenzol, Dichlorbenzol (ortho oder para oder Isomerengemische daraus), Trichlorbenzol, 1,3,5-Trimethylbenzol (Mesitylen), Xylol, Hexan, Diethylisophthalat (DEIP), Tetrahydrofuran (THF), Dimethylformamid (DMF) und deren Gemische, bevorzugt Monochlorbenzol.

In einer besonderen Ausführungsform des erfindungsgemäßen Verfahrens handelt es sich bei der eingedüsten Flüssigkeit um ein Gemisch aus Isocyanaten, ein Gemisch aus Isocyanaten und Lösungsmittel oder um Isocyanat, wobei die jeweils verwendete Quenchflüssigkeit Anteile an Leichtsieder, wie Chlorwasserstoff und Phosgen, aufweisen kann. Vorzugsweise wird dabei das Isocyanat eingesetzt, das bei dem jeweiligen Verfahren hergestellt wird. Durch die Temperatursenkung in der Quenchzone können Nebenreaktionen mit den eingedüsten Isocyanaten ausgeschlossen werden. Der Vorteil dieser Ausführungsform liegt insbesondere darin, dass auf eine Abtrennung des Lösungsmittels verzichtet werden kann.

In einer alternativen bevorzugten Ausführungsform handelt es sich bei dem Inertmedium, das zusammen mit mindestens einem der Edukte eingesetzt wird, und bei dem Lösungsmittel, das im Quench eingesetzt wird, um die gleiche Verbindung. Ganz besonders bevorzugt wird in diesem Fall Monochlorbenzol verwendet.

Im Rahmen von Schritt (bb) wird das Reaktionsgemisch, das im Wesentlichen aus den Isocyanaten, Phosgen und Chlorwasserstoff besteht, intensiv mit der eingedüsten Flüssigkeit vermischt. Die Vermischung erfolgt derart, dass die Temperatur des Reaktionsgemisches ausgehend von 150 bis 250°C auf 100 bis 200 °C, bevorzugt auf 100 bis 160 °C abgesenkt wird und das im Reaktionsgemisch enthaltene Isocyanat durch Kondensation vollständig oder teilweise in die eingedüsten Flüssigkeitströpfchen übergeht, während das Phosgen und der Chlorwasserstoff im wesentlichen vollständig in der Gasphase verbleiben.

Der Anteil des im gasförmigen Reaktionsgemisch enthaltenen Isocyanats, das vorzugsweise in der Quenchzone in die Flüssigphase übergeht, beträgt dabei vorzugsweise 20 bis 100 Gew.-%, besonders bevorzugt 50 bis 99,5 Gew.-% und insbesondere 70 bis 99 Gew.-%, bezogen auf das gesamte im Reaktionsgemisch enthaltene Isocyanat.

Der Anteil des im gasförmigen Reaktionsgemisch enthaltenen Phosgens und Chlorwasserstoffs, das in der Quenchzone in die Flüssigphase übergeht, beträgt dabei in der Regel weniger als 25 Gew.-%, vorzugsweise weniger als 10 Gew.-%.

Um die Absorption von Phosgen in der Quenchflüssigkeit erfindungsgemäß niedrig zu halten wird der Quench bevorzugt bei niedrigem Druck, beispielsweise 1 bis 5 bar, und erhöhter Temperatur, beispielsweise 100 bis 160 °C, durchgeführt.

Besonders bevorzugt wird dabei die Temperatur des inerten Lösungsmittels oberhalb der Lösungstemperatur des zum Amin gehörigen Carbamoylchlorids im gewählten Quenchmedium gehalten.

Grundsätzlich können für diese Verfahrensstufe alle an sich bekannten und für Absorptionen geeignete Methoden und Apparate eingesetzt werden, beispielsweise eine Waschkolonne oder das Sprühquenchverfahren.. Das Quenching kann ein- oder mehrstufig, bevorzugt einstufig durchgeführt werden. Das Quenching kann darüber hinaus in Abhängigkeit von dem verwendeten Verfahren in Gleich- oder Gegenstromfahrweise realisiert werden.

Ein weiterer geeigneter Quench ist beispielsweise bekannt aus EP-A 1 403 248, Sp. 2, Z. 39 - Sp. 3, Z. 18, auf die im Umfang dieser Offenbarung ausdrücklich Bezug genommen sei. Des Weiteren kann das Quenching gemäß den Ausführungen in WO 2008/055899, WO 2008/055904 oder WO 2005/123665 ausgeführt werden, auf deren Inhalt hiermit in vollem Umfang Bezug genommen sei.

Das Reaktionsgemisch durchströmt die Quenchzone vorzugsweise von oben nach unten. Unterhalb der Quenchzone ist ein Sammelbehälter angeordnet, in dem die Flüssigphase abgeschieden, gesammelt und über einen Auslass entfernt und anschließend aufgearbeitet wird. Die verbleibende Gasphase wird über einen zweiten Auslass aus dem Sammelbehälter entfernt und ebenfalls aufgearbeitet.

Geringe Mengen von Nebenprodukten, die im Isocyanat verbleiben, können insbesondere mittels zusätzlicher Rektifikation, durch Strippen mit einem Inertgas oder durch Kristallisation, bevorzugt durch Rektifikation, vom erwünschten Isocyanat getrennt werden.

### Aufarbeitung

In Anschluss an Schritt (b) und gegebenenfalls weiterer Kühlstufen (bb usw.) erfolgt optional im Rahmen eines Schrittes (c) die Aufarbeitung des Isocyanats unter Entfernung von Nebenprodukten und nicht reagierter Edukte, was insbesondere die Verdampfung des in der kondensierten Phase verbliebenen Phosgens und Chlorwasserstoffs einschließt.

Entsprechende Verfahren zur Aufarbeitung des Isocyanats sind dem Fachmann bekannt. Der Fachmann wählt ein oder mehrere geeignete Aufarbeitungsverfahren in Abhängigkeit von der Temperatur und dem Phasenzustand der Reaktionsprodukte, der auch von der Art der Kühlung abhängt, die zuletzt angewendet wurde.

Im Rahmen einer bevorzugten Ausführungsform wird das Isocyanat durch Destillation und besonders bevorzugt durch Rektifikation vom Lösungsmittel abgetrennt. Ebenfalls kann hier noch die Abtrennung von restlichen Verunreinigungen, umfassend Chlorwasserstoff, Inertmedium und/oder Phosgen, erfolgen, wie beispielsweise beschrieben in DE-A 10260092.

Im Rahmen von Schritt (c) werden gasförmige Stoffströme, die im Wesentlichen aus Phosgen und/oder Chlorwasserstoffgas sowie gegebenenfalls Inertmedium bestehen, erhalten. Aus zumindest einem Teil dieser Phosgen und/oder Chlorwasserstoffgas und/oder Inertmedium enthaltenden Stoffströme wird in dem Fachmann bekannter Weise Chlorwasserstoff vom Phosgen abgetrennt.

In einer weiteren bevorzugten Ausführungsform erfolgt die Abtrennung so, dass der Massenbruch von Chlorwasserstoff im phosgenhaltigen Strom nach gegebenenfalls durchgeführter Vermischung mit Frisch-Phosgen vor der Vermischung mit dem aminhaltigen Strom weniger als 15 Gew.-%, bevorzugt weniger als 10, besonders bevorzugt weniger als 5 Gew.-% beträgt.

Die Trennung des Chlorwasserstoff und/oder Phosgen sowie gegebenenfalls Lösungsmittel aus dem Quench enthaltenden Gemisches erfolgt bevorzugt über Destillation und/oder eine Wäsche. Bevorzugt wird die Trennung in einer Kombination von einer Destillation und einer Wäsche durchgeführt.

Beim Waschmedium kann es sich insbesondere um Toluol, Benzol, Nitrobenzol, Anisol, Chlorbenzol, Dichlorbenzol (ortho oder para oder Isomerengemische daraus), Trichlorbenzol, Xylol, Hexan, Diethylisophthalat (DEIP), Tetrahydrofuran (THF), Dimethylformamid (DMF) und deren Gemische, bevorzugt Monochlorbenzol handeln. Besonders bevorzugt wird als Waschflüssigkeit das gleiche Lösungsmittel eingesetzt, wie in Schritt (bb) beim Quenching. Die Wäsche kann im Gleichstrom oder bevorzugt im Gegenstrom erfolgen.

Bei einer kombinierten Wäsche und Destillation, beispielsweise einer Druckdestillation, wird Phosgen aus dem chlorwasserstoffhaltigen Strom durch Wäsche mit einem Waschmedium, bevorzugt Toluol, Chlorbenzol oder Dichlorbenzol, ausgewaschen.

Hier liegt ausnahmsweise Phosgen in kondensierter Form vor. Die Abtrennung von Phosgen und Chlorwasserstoff aus dem beladenen Waschmedium nach der Wäsche erfolgt bevorzugt destillativ oder durch Desorption. Dabei erfolgt die Abtrennung bevorzugt so, dass ein gasförmiger Phosgenstrom anfällt, der in einer besonders bevorzugten Ausführungsform, gegebenenfalls nach Vermischung mit Frischphosgen, einen Gehalt an Chlorwasserstoff von kleiner 15 Gew.-% enthält.

Die Wäsche und die Destillation werden bei Drücken von 1 bis 10 bar absolut betrieben, bevorzugt von 1 bis 5 bar absolut.

Der Chlörwasserstoff-/Phosgentrennung kann eine Adsorptionseinheit, bevorzugt ein Aktivkohlefilter, im aus der Trennung abgeführten Chlorwasserstoffsstrom nachgeschaltet werden, in dem Spuren des Waschmediums aus dem anfallenden Chlorwasserstoff entfernt werden.

Anschließend erfolgt in einer optionalen Ausführungsform die Rückführung des so erhaltenen überwiegend Phosgen enthaltenden Stromes als Einsatzstoff im erfindungsgemäßen Verfahren.

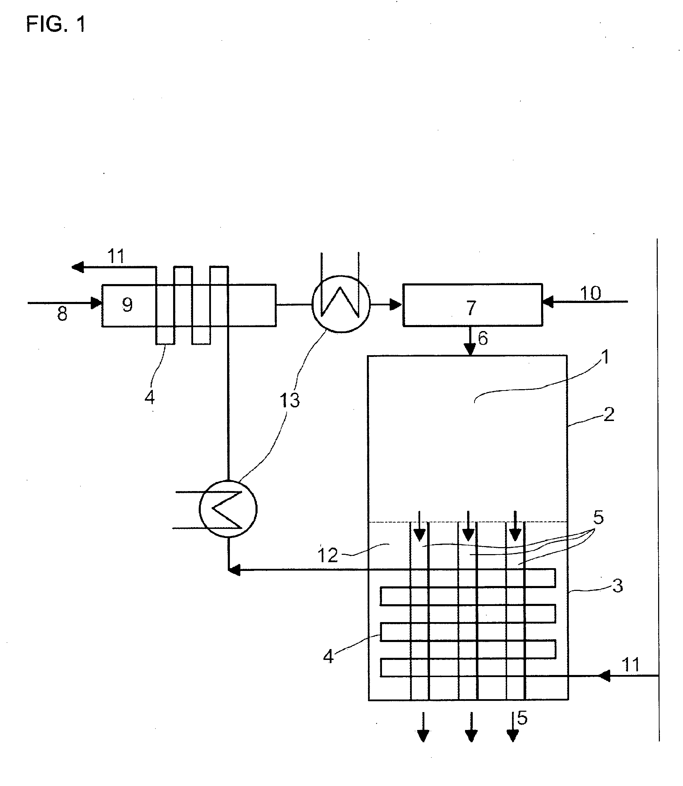

Eine bevorzugte Ausführungsform der vorliegenden Erfindung ist in Abbildung 1 schematisch veranschaulicht. Abbildung 1 dient dabei der Erläuterung dieser bevorzugten Ausführungsform der vorliegenden Erfindung und ist nicht einschränkend zu verstehen. Einzelne Elemente der nachfolgend erläuterten bevorzugten Ausführungsform können vorteilhaft mit oben erläuterten Ausführungsformen kombiniert werden.

In Abbildung 1 bedeutet:
1 Reaktionszone
2 Reaktor
3 Kühlvorrichtung
4 Kühlfluidführung
5 Produktstrom
6 Eduktstrom
7 Mischvorrichtung
8 Aminstrom
9 Aufwärmvorrichtung
10 Phosgenstrom
11 Kühlstrom
12 Kühlzone
13 Optionale ergänzende Wärmequelle

### Beschreibung der bevorzugten Ausführungsform gemäß Abbildung 1:

Der Eduktstrom (6) umfassend mindestens ein Amin und Phosgen tritt in die Reaktionszone (1) ein, die sich innerhalb eines Reaktors (2) befindet. Anschließend tritt der resultierende Gasstrom (Produktstrom (5)) in die Kühlzone (12) ein, die sich innerhalb der Kühlvorrichtung (3) befindet, die vorzugsweise ein Rohrbündelwärmeübertrager (Rohrbündelwärmetauscher) ist.

Die Kühlung des Produktstromes (5) erfolgt mittels eines Kühlstromes (11), der in Gegenstromrichtung zur Richtung des Produktstromes (5) innerhalb der Kühlfluidführung (4) fließt, wobei die Kühlfluidführung (4) innerhalb der Kühlvorrichtung (3) vorzugsweise in Form von Umlenkvorrichtungen innerhalb eines Rohrbündelwärmeübertragers, insbesondere Umlenkbleche, realisiert wird. In der bevorzugten Ausführungsform kreuzt der Kühlstrom den Produktstrom an mehreren Stellen (gekreuzte Ausführung, siehe oben). Der Produktstrom (5) verlässt anschließend den Bereich der indirekten Kühlung. Der erhitzte Kühlstrom (11) wird gegebenenfalls mittels einer optionalen ergänzenden Wärmequelle (13) weiter erhitzt und wird anschließend zur Erwärmung des Aminstromes (8) in einer Aufwärmvorrichtung (9) verwendet. Die optionale ergänzenden Wärmequelle (13) kann insbesondere eine elektrische Heizvorrichtung sein oder ein Wärmetauscher, der mit Brenngasen operiert.

Der erhitzte Aminstrom (8) verlässt die Aufwärmvorrichtung (9) und wird mittels einer optionalen ergänzenden Wärmequelle (13) weiter erhitzt. Die nach Vorerhitzung des Aminstromes (8) gegebenenfalls implementierte optionale ergänzende Wärmequelle (13) kann wie oben erläutert insbesondere als elektrische Heizvorrichtung oder als Wärmetauscher, der mit Brenngasen operiert, ausgelegt werden. Anschließend tritt der vorerhitzte Aminstrom (8) ebenso wie der Phosgenstrom (10) in die Mischvorrichtung (7) ein. Die Mischvorrichtung (7) kann eine vom Reaktor (2) separierte Vorrichtung sein oder in den Reaktor (2) integriert sein.

## Patentansprüche

1. Verfahren zur Herstellung von Isocyanaten umfassend
(a) die Umsetzung von mindestens einem Amin mit Phosgen in der Gasphase in einer Reaktionszone und
(b) die anschließende Abkühlung der Reaktionsgase in einer Kühlzone mittels indirekter Kühlung, wobei das die Wärme der Reaktionsgase aufnehmende Kühlmedium zumindest im Bereich der höchsten Temperatur in der Kühlzone im Gegenstrom zum Produktstrom geführt wird.

2. Verfahren nach Anspruch 1, wobei die Abkühlung gemäß Schritt (b) in einem Wärmetauscher mit einer spezifischen Wärmeübertragungsfläche von mindestens 300 m² pro m³ Reaktionsgas durchgeführt wird.

3. Verfahren nach Anspruch 2, wobei der Wärmetauscher ein Rohrbündelwärmetauscher ist.

4. Verfahren nach einem oder mehreren der Ansprüche 1 bis 3, wobei die Abkühlzeit gemäß Schritt (b) von 0,02 bis 5 Sekunden beträgt.

5. Verfahren nach einem oder mehreren der Ansprüche 1 bis 4, wobei das Isocyanat im Rahmen von Schritt (b) nicht kondensiert vorliegt.

6. Verfahren nach einem oder mehreren der Ansprüche 1 bis 5, wobei sich die Reaktionszone gemäß Schritt (a) und die Kühlzone gemäß Schritt (b) innerhalb eines Apparates befinden.

7. Verfahren nach einem oder mehreren der Ansprüche 1 bis 6, wobei in Anschluss an die Kühlung gemäß Schritt (b) als Schritt (bb) eine weitere Abkühlung der Reaktionsgase erfolgt.

8. Verfahren nach Anspruch 7, wobei die Kühlung gemäß Schritt (b) und (bb) jeweils indirekt durchgeführt wird und wobei sich die Kühlmedien gemäß Schritt (b) und (bb) in unterschiedlichen Kreisläufen befinden.

9. Verfahren nach Anspruch 7, wobei im Rahmen von Schritt (bb) eine direkte Kühlung der Reaktionsgase erfolgt.

10. Verfahren nach Anspruch 9, wobei im Rahmen von Schritt (bb) mittels Quenching gekühlt wird.

11. Verfahren nach einem oder mehreren der Ansprüche 1 bis 10, wobei die im Rahmen von Schritt (b) vom Kühlmedium aufgenommene Wärme mindestens teilweise zur indirekten Wärmeübertragung auf mindestens einen Eduktstrom der Umsetzung gemäß Schritt (a) genutzt wird.

12. Verfahren nach einem oder mehreren der Ansprüche 1 bis 11, wobei das die Wärme der Reaktionsgase aufnehmende Kühlmedium eine Salzschmelze ist.

13. Verfahren nach einem oder mehreren der Ansprüche 1 bis 12, wobei als Amin mindestens ein aromatisches Diamin mit von 6 bis 18 Kohlenstoffatomen oder mindestens ein (cyclo)aliphatisches Diamin mit von 2 bis 18 Kohlenstoffatomen verwendet wird.

14. Verfahren nach einem oder mehreren der Ansprüche 1 bis 13, wobei als Isocyanat Toluylendiisocyanat hergestellt wird.

15. Verfahren nach einem oder mehreren der Ansprüche 1 bis 13, wobei als Isocyanat mindestens eines ausgewählt aus Diisocyanatodiphenylmethanen hergestellt wird.

16. Verfahren nach einem oder mehreren der Ansprüche 1 bis 13, wobei als Isocyanat Hexamethylendiisocyanat hergestellt wird.

## Claims

1. A process for preparing isocyanates, comprising
(a) the reaction of at least one amine with phosgene in the gas phase in a reaction zone and
(b) the subsequent cooling of the reaction gases in a cooling zone by means of indirect cooling, the cooling medium which absorbs the heat of the reaction gases being conducted in countercurrent to the product stream at least in the region of the highest temperature in the cooling zone.

2. The process according to claim 1, wherein the cooling in step (b) is performed in a heat exchanger with a specific heat transfer area of at least 300 m² per m³ of reaction gas.

3. The process according to claim 2, wherein the heat exchanger is a tube bundle heat exchanger.

4. The process according to one or more of claims 1 to 3, wherein the cooling time in step (b) is from 0.02 to 5 seconds.

5. The process according to one or more of claims 1 to 4, wherein the isocyanate is not present in condensed form in the course of step (b).

6. The process according to one or more of claims 1 to 5, wherein the reaction zone in step (a) and the cooling zone in step (b) are within one apparatus.

7. The process according to one or more of claims 1 to 6, wherein the cooling in step (b) is followed, as step (bb), by a further cooling of the reaction gases.

8. The process according to claim 7, wherein the cooling in steps (b) and (bb) is in each case performed indirectly, and wherein the cooling media in steps (b) and (bb) are within different circuits.

9. The process according to claim 7, wherein the reaction gases are cooled directly in step (bb).

10. The process according to claim 9, wherein cooling is effected by means of quenching in step (bb).

11. The process according to one or more of claims 1 to 10, wherein the heat absorbed by the cooling medium in step (b) is utilized at least partly for indirect heat transfer to at least one reactant stream of the reaction in step (a).

12. The process according to one or more of claims 1 to 11, wherein the cooling medium which absorbs the heat of the reaction gases is a salt melt.

13. The process according to one or more of claims 1 to 12, wherein the amine used is at least one aromatic diamine having from 6 to 18 carbon atoms or at least one (cyclo)aliphatic diamine having from 2 to 18 carbon atoms.

14. The process according to one or more of claims 1 to 13, wherein the isocyanate prepared is tolylene diisocyanate.

15. The process according to one or more of claims 1 to 13, wherein the isocyanate prepared is at least one selected from diisocyanatodiphenylmethanes.

16. The process according to one or more of claims 1 to 13, wherein the isocyanate prepared is hexamethylene diisocyanate.

## Revendications

1. Procédé pour la préparation d'isocyanates, comprenant
(a) la transformation d'au moins une amine avec du phosgène en phase gazeuse dans une zone de réaction et
(b) le refroidissement consécutif des gaz de réaction dans une zone de refroidissement par refroidissement indirect, l'agent de refroidissement absorbant la chaleur des gaz de réaction étant guidé, au moins dans la zone de la température la plus élevée dans la zone de refroidissement, à contre-courant par rapport au flux de produits.

2. Procédé selon la revendication 1, le refroidissement selon l'étape (b) étant réalisé dans un échangeur thermique présentant une surface spécifique de transfert de chaleur d' au moins 300 m² par m³ de gaz de réaction.

3. Procédé selon la revendication 2, l'échangeur thermique étant un échangeur thermique à faisceau tubulaire.

4. Procédé selon l'une ou plusieurs des revendications 1 à 3, le temps de refroidissement selon l'étape (b) étant de 0,02 à 5 secondes.

5. Procédé selon l'une ou plusieurs des revendications 1 à 4, l'isocyanate se trouvant sous forme non condensée dans le cadre de l'étape (b).

6. Procédé selon l'une ou plusieurs des revendications 1 à 5, la zone de réaction selon l'étape (a) et la zone de refroidissement selon l'étape (b) se trouvant au sein d'un appareil.

7. Procédé selon l'une ou plusieurs des revendications 1 à 6, un autre refroidissement des gaz de réaction ayant lieu en tant qu'étape (bb) consécutivement au refroidissement selon l'étape (b).

8. Procédé selon la revendication 7, le refroidissement selon les étapes (b) et (bb) étant réalisé à chaque fois indirectement et les agents de refroidissement selon les étapes (b) et (bb) se trouvant dans des circuits différents.

9. Procédé selon la revendication 7, un refroidissement direct des gaz de réaction ayant lieu dans le cadre de l'étape (bb).

10. Procédé selon la revendication 9, le refroidissement dans le cadre de l'étape (bb) étant un refroidissement rapide.

11. Procédé selon l'une ou plusieurs des revendications 1 à 10, la chaleur absorbée par l'agent de refroidissement dans le cadre de l'étape (b) étant utilisée au moins partiellement pour le transfert de chaleur indirect à au moins un flux de produits de départ de la transformation selon l'étape (a).

12. Procédé selon l'une ou plusieurs des revendications 1 à 11, l'agent de refroidissement absorbant la chaleur des gaz de réaction étant un sel fondu.

13. Procédé selon l'une ou plusieurs des revendications 1 à 12, au moins une diamine aromatique comprenant 6 à 18 atomes de carbone ou au moins une diamine (cyclo)aliphatique comprenant 2 à 18 atomes de carbone étant utilisée comme amine.

14. Procédé selon l'une ou plusieurs des revendications 1 à 13, du diisocyanate de toluylène étant préparé comme isocyanate.

15. Procédé selon l'une ou plusieurs des revendications 1 à 13, au moins un isocyanate choisi parmi les diisocyanatodiphénylméthanes étant préparé comme isocyanate.

16. Procédé selon l'une ou plusieurs des revendications 1 à 13, du diisocyanate d'hexaméthylène étant préparé comme isocyanate.
